# EUROPEAN PATENT APPLICATION

(11) **EP 1 462 061 A2**
(43) Date of publication of application: **29.09.2004**
(21) Application number: 04251566.8
(22) Date of filing: 18.03.2004
(51) Int. Cl.: A61B 17/122

(54) **Passive surgical clip**

(30) Priority: 25.03.2003 US 396240
(71) Applicant: ETHICON ENDO-SURGERY, INC., Cincinnati, Ohio 45242 (US)
(72) Inventor: Kortenbach, Juergen A., Miami Springs FL 33166 (US); Smith, Kevin W., Coral Gables FL 33156 (US); Francese, Jose Luis, Miami Springs FL 33166 (US); Rivera, Carlos M., Cooper City FL 33330 (US)
(74) Representative: Tunstall, Christopher Stephen

(57) **Abstract**

A clip includes a generally U-shaped configuration with first (412) and second (414) parallel arms and a bridge (416) connecting the arms. At least one resilient retainer (422) extends rearward and from at least one of the arms and toward the other arm. During application, the clip is forced over compressed tissue. As the clip is forced over the tissue and the tissue enters the space between the arms, the retainers are bent by the tissue. The retainer is preferably resilient, but may alternatively be plastically deformable. The clip is also provided with structure (425,432) that facilitates the chaining of a plurality of clips for use in a clip chamber of a clip applier.

## Description

### BACKGROUND OF THE INVENTION

### 1. Field of the Invention

This invention relates broadly to surgical devices. More particularly, this invention relates to a surgical clip for clamping and/or suturing, ducts, vessels, and other tissues, for anchoring a tissue, or for attaching a foreign body to a tissue.

### 2. State of the Art

Surgical clips are generally used to apply clamping force to ducts, vessels, and other tissues. In addition, surgical clips are particularly useful in controlling bleeding of a tissue in lieu of suturing or stapling where suturing or stapling is difficult. However, in certain circumstances, the bleeding tissue is lubricous, and applied clips often slip from the tissue and are dislodged, removing the necessary clamping force thereabout. This is particularly a problem when a clip is provided about tissue which is not a conduit of a size which can be completely surrounded by the clip. For example, it is very difficult to secure a clip about a small peripheral portion of ulcerated stomach tissue and therefore it is difficult to effect hemostasis of such bleeding tissue with a clip. Moreover, the problem is amplified when the clip used is very small.

In order to prevent dislodgement, a combination of a clip and a staple has been described in U.S. Patent No. 5,522,823 to Kuntz et al. In the Kuntz clip, one end portion of the clip is pierced through the tissue and captured in an eye of another end portion of the clip to secure the clip on the tissue. With the clip piercing the tissue, the likelihood that the clip will become inadvertently dislodged is greatly reduced.

While the Kuntz et al. clip represents a step forward, the disclosed clip is not particularly useful in endoscopic procedures. In particular, both the nature of the clip and the manner in which it is applied are complex. For example, in order to facilitate the bending of the clip through various configurations required of its applier, the clip has portions provided with at least four different widths as well as an eye opening. This complex clip structure is not practical for a clip which is to be used in a flexible endoscopy procedure in which the tools used are of very small diameter, e.g., 2-6 mm (0.08 - 0.24 inch). In addition, for endoscopic procedures it is highly desirable that multiple clips be able to be applied without removing the clip applier from its general location. The Kuntz et al. clip and applier, however, are not particularly adapted for applying multiple clips, as the Kuntz et al. clip does not stack, and the applier with which it is used holds a single clip at a time.

### SUMMARY OF THE INVENTION

It is therefore an object of the invention to provide a surgical clip which remains secured to the tissue to which it is applied.

It is another object of the invention to provide a surgical clip which is passively applied to tissue; i.e., without undergoing plastic deformation.

It is a further object of the invention to provide a surgical clip which is adapted for use in minimally invasive surgery.

It is an additional object of the invention to provide a surgical clip which can be applied in a flexible endoscopy setting.

It is also an object of the invention to provide a surgical clip which can be used with rigid instruments operated through a port in the human body.

It is yet another object of the invention to provide a surgical clip which can be used in open surgery.

It is still a further object of the invention to provide a surgical clip which is relatively easy to manufacture.

It is still another object of the invention to provide a surgical clip which is particularly adapted for use in an applier which holds a plurality of clips.

In accord with these objects, which will be discussed in detail below, a surgical clip includes a generally U-shaped configuration with first and second parallel arms and a bridge portion connecting the arms.

According to several embodiments of the clip, the first arm narrows opposite the bridge portion, and forms a preferably resilient retainer which extends rearward, back into a space between the arms, and also toward the second arm. The retainer may be straight or curved in a direction toward or away from the first arm. The retainer includes a tip at which a barb is preferably provided. The second arm may also include a recess toward which or into which the tip of the retainer is directed.

According to another embodiment of the clip, two resilient retainers are provided, each extending from the first arm. Preferably, one retainer extends rearward as described above, while a second shorter retainer extends toward a free end of the second arm. The shorter retainer may be resilient. Alternatively, the shorter retainer may sized, shaped or otherwise constructed for plastic deformation. Furthermore, two or more longitudinally offset retainers on the same arm may extend rearward into the space between the arms.

According to other embodiments, two retainers are provided, one on each of the first and second arms. Both retainers extend from narrowed portions of the leading ends of the arms and are directed rearward into the space between the arms.

According to further embodiments of the clip, a plurality of rearward-facing retainers are provided on each of the first and second arms. These retainers are preferably interleaved so that the retainers may be relatively long without interfering with generally opposing retainers. The retainers are preferably substantially resilient, but may alternatively be adapted for plastic deformation.

During application, the clip is forced over compressed tissue. As the clip is forced over the tissue and the tissue enters the space between the arms, the tissue resiliently or plastically bends the retainers, according to the retainer construction. Once the clip is provided over the tissue, it is retained thereover, as the rearward direction of the retainer prevents release of the clip from the tissue.

According to another preferred aspect of all embodiments of the clip, the clip is provided with structure that facilitates the stacking (or chaining) of a plurality of clips in a clip chamber of an applier. To that end, the leading end and rear bridge portions of the clip are adapted for stacking and, in some embodiments, mechanically interlocking with adjacent clips. The bridge portion of a relatively distal clip fits at least partially into a space between the first and second arms at the leading end of a relatively proximal clip. During deployment of a distalmost clip, the ends of the first and second arms of a relatively proximal are permitted to move apart to release the distalmost clip from the chain of clips.

Additional objects and advantages of the invention will become apparent to those skilled in the art upon reference to the detailed description taken in conjunction with the provided figures.

### BRIEF DESCRIPTION OF THE DRAWINGS

Fig. 1 is a perspective view of a train of a plurality of surgical clips according to a first embodiment of the invention;
Fig. 2 is a side elevation of a second embodiment of a surgical clip according to the invention;
Fig. 3 is a side elevation of a third embodiment of a surgical clip according to the invention;
Fig. 4 is a side elevation of a fourth embodiment of a surgical clip according to the invention;
Fig. 5 is a side elevation of a fifth embodiment of a surgical clip according to the invention;
Fig. 6 is a side elevation of a sixth embodiment of a surgical clip according to the invention;
Fig. 7 is a side elevation of a seventh embodiment of a surgical clip according to the invention;
Fig. 8 is a side elevation of an eighth embodiment of a surgical clip according to the invention; and
Fig. 9 is a side elevation of a ninth embodiment of a surgical clip according to the invention.

### DETAILED DESCRIPTION OF THE PREFERRED EMBODIMENTS

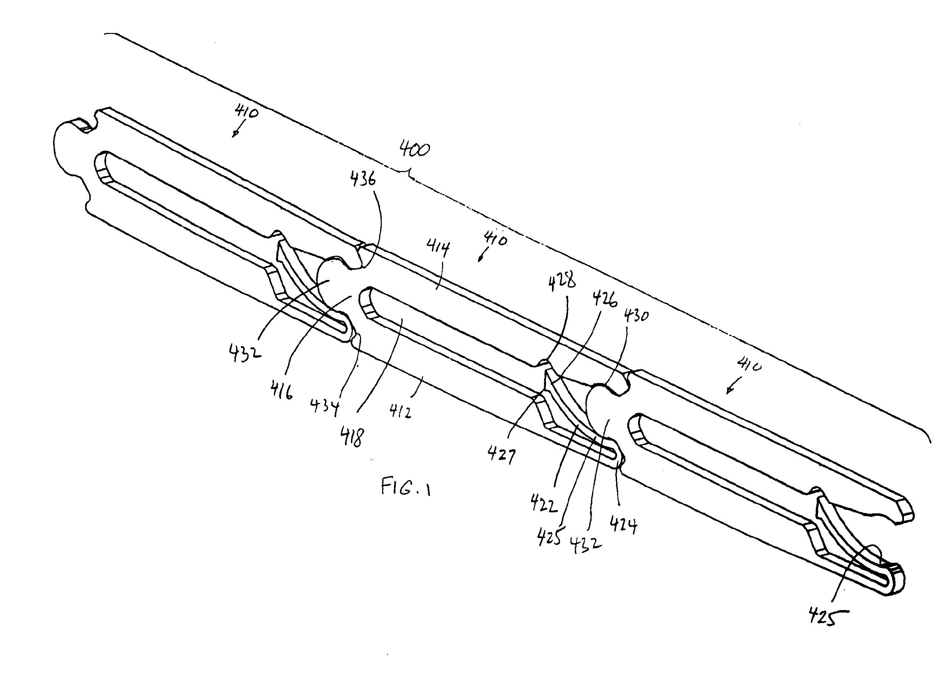

Turning now to Fig. 1, a train 400 of three surgical clips 410 is shown. Such a train 400 may include only two clips or more than three clips. Each clip 410 includes first and second substantially parallel arms 412, 414, and a bridge portion 416 therebetween such that the arms and bridge portion are in a generally U-shaped configuration with a space 418 defined therebetween.

The first arm 412 extends (or transitions) into a resilient retainer 422 which has a smaller thickness than the first arm 412. Retainer 422 turns through slightly more than 180° at a distalmost extension 424 of the arm, and then curves upward toward the second arm 412. By curving through more than 180°, this portion of the retainer defines a first catch portion 425. The retainer 422 preferably terminates in a barbed tip 426 with the barb 427 preferably facing toward the first arm 412. The retainer preferably interrupts access to the space 418 between the arms from a leading end of the clip. The second arm 414 defines a notch 428 towards which the tip 426 is directed and may be partially received, and a relatively distal second catch portion 430. The bridge portion 416 includes a generally omega-shaped projection 432 and recesses 434, 436 defined between the projection 432 and the first arm 412 and between the projection 432 and the second arm 414.

The projection 432, recesses 434, 436, the first catch portion 425 of the first arm 412, and the second catch portion 430 of the second arm 414 cooperate to define a mechanical interlock such that a plurality of clips 410 may be coupled together in a linear arrangement to define the train 400. That is, when longitudinally arranged, the projection 432 of a relatively distal clip extends between the first and second arms 412, 414 and into the catch portions 425, 430 thereof, with the end of the first arm 412 extending around the projection and into recess 434, while the end of the second arm 414 extends around the projection 432 and into recess 436. When the clips 410 are provided in a clip chamber of a clip applier instrument and such chamber is sized or otherwise configured to restrict movement of the first and second arms away from each, the projection 432 of a relatively distal clip is captured and retained by the adjacent relatively proximal clip. As such, the clips do not separate from each other while in a clip chamber.

In use, a clip chamber of a clip applier (not shown) is loaded with a train of the clips 410. The clip applier includes a distal end having a jaw assembly (not shown) and an exit through which clips can be advanced from the clip chamber to between the jaws. Flexible endoscopic clip appliers having such features are generally disclosed in co-pending U.S. Serial Nos. 09/891,775, filed June 25, 2001, and 10/010,906, filed Dec. 6, 2001, which are hereby incorporated by reference herein in their entireties. The clip applier is inserted through an endoscope into the human body, and the jaw assembly is moved adjacent the target tissue identified for receiving a clip and operated to clamp and compress the target tissue. Once the tissue is compressed, preferably to a thickness approximating the space 418 between the first and second arms 412, 414, the distalmost clip of the clip train 400 is advanced through the jaw assembly and over the compressed tissue. The retainer 422 is resiliently deformed during advancement such that the retainer is bent to provide access to the space 418. The clip is advanced over the tissue, preferably until the tissue is either seated against the bridge 416 of the clip and/or fully resides proximal of the end 426 of the retainer 422 so that the retainer may reform to again interrupt the space. The arms 412, 414 and bridge 416 are relatively stiff, such that the arms and bridge retain their shape and are not plastically deformed during application over tissue. That is, any expansion of the clip between the arms during application is minimal and elastic. In addition, it is appreciated that the clip is passive in that the clip is not actively deformed by the clip applier during application.

Regardless of the exact position of the clip on the tissue, movement of the clip 410 in a release direction is prohibited by the retainer 422 which is directed to pierce or contact tissue if the clip is urged in a release direction. After attaching the clip to tissue, the jaw assembly is opened to remove clamping force from the tissue.

When the distalmost clip is attached to the tissue, the jaw assembly may be arranged such that the end of the arms of the proximally adjacent clip (i.e., the next clip) extend partially out of the clip chamber and into the jaw assembly. Therefore, the end of the arms of the proximally adjacent clip may not be constrained by the clip chamber. As such, after the distalmost clip is attached to tissue and the jaw assembly is opened, the clip applier (or just the clip train) can be retracted to subject the clip train 400 to a tensile force. The tensile force urges the unconstrained arms of the clip proximally adjacent to the distalmost clip to slightly flex and thereby release the projection 432 of the distalmost clip to deploy the clip. The clip train may then be slightly retracted in preparation of the application of the next clip.

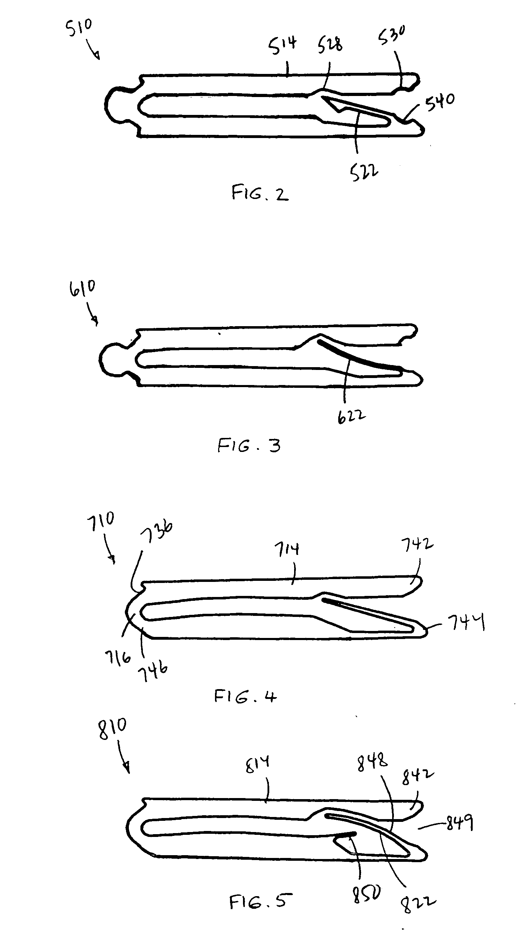

Referring now to Fig. 2, a second embodiment of a clip 510 is shown. In the second embodiment and each of subsequent embodiments, like parts to the first embodiment have reference numerals which are incremented by 100 for each ordinal embodiment. The second embodiment 510 is substantially similar to the first embodiment, with three distinctions. First, the end of the first arm 512 is provided with a catch 540 opposite and similar to a catch 530 at the end of the second arm 514. That is, catch 540 is not part of the retainer 522. Second, the retainer 522 does not extend through a tight turn at the end of the first arm, but rather extends from a location proximally adjacent the catch 540. Third, the retainer 522 extends substantially straight, diagonally rearward and upward, toward a notch 528 in the second arm 514.

Referring now to Fig. 3, a third embodiment of a clip 610, substantially similar to the first and second embodiments, is shown. The clip 610 includes a non-barbed retainer 622.

Referring now to Fig. 4, a fourth embodiment of a clip 710 is shown. Clip 710 is adapted to stack with adjacent clips, but does not include structure permitting the clips to be interlocked in a train. Stacking of the clips is facilitated, in that the rear of the bridge 716 includes an upper notch 736 shaped and sized to receive the end 742 of a second arm 714 of a proximally adjacent clip, and the end 744 of the first arm 712 is adapted to fit about the lower portion 746 of the bridge 716. In addition, the retainer 722 of clip 710 extends upward and rearward from the end 744 of the first arm.

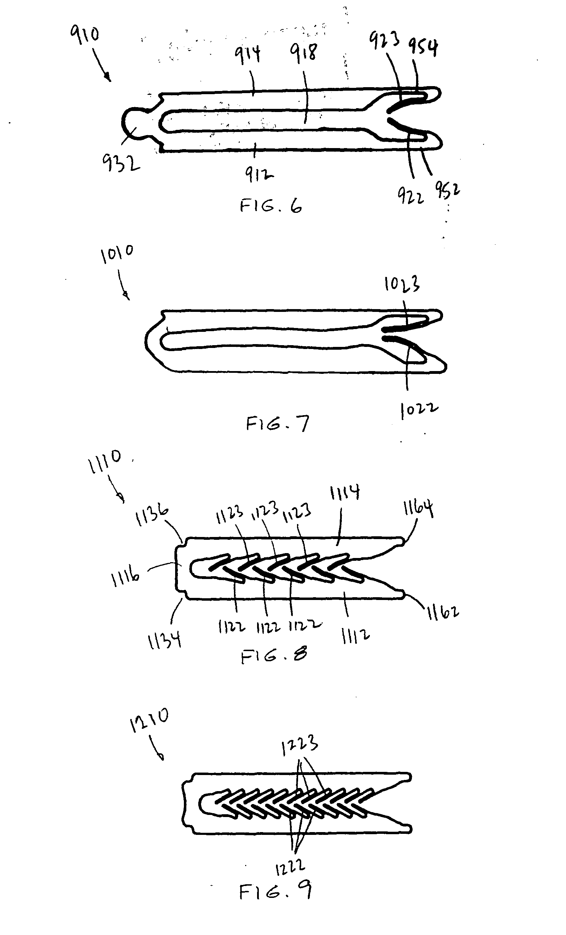

Referring now to Fig. 5, a fifth embodiment of a clip 810 is shown. The clip 810 is substantially similar to clip 710 with two distinctions. First, the main or first retainer 822 curves toward the second arm 814 such as to present a convex face 848 at the entry or ingress 849 of the clip 822. Second, a relatively shorter second retainer 850 extends from the first arm 812. The shorter retainer 850 preferably extends toward a free end 842 of the second arm 814. The shorter retainer 850 may be resilient, or alternatively dimensioned or otherwise structured for plastic deformation.

Referring now to Fig. 6, a sixth embodiment of a clip 910 is shown. The clip 910 includes two retainers 922, 923, one on each of the first and second arms 912, 914. Both retainers 922, 923 extend from narrowed portions 952, 954 of the leading ends of the arms 912, 914 and are directed rearward into the space 918 between the arms. The retainers 922, 923 are curved to present concave surfaces at the entry to the space 918 between the arms. Such configuration permits the retainers 922, 923 to form around and hold onto a projection 932 on a relatively distal clip to link the clips together. Alternatively, the retainers may be otherwise formed to present a convex surface (shown in Fig. 7 with respect to retainers 1022 and 1023 on a seventh embodiment of a clip 1010), or relatively straight surfaces such that together they generally define a V. Where the retainers 1022, 1023 extend in accord with the alternate configurations (i.e., do not present concave surfaces), an alternate stacking mechanism, such as discussed with respect to Fig. 4, which does not rely on interlocking of the clips, can be used.

Turning now to Fig. 8, an eighth embodiment of a clip 1110 according to the invention is shown. The clip 1110 includes a plurality of relatively short rearward-facing retainers 1122, 1123 on each of the first and second arms 1112, 1114. Retainers 1122 on the first arm 1112 are preferably interleaved with retainers 1123 on the second arm 1114 to prevent interference between the retainers on the first and second arms. The retainers 1122, 1123 are preferably spaced-apart along a substantial length of each of the arms. Referring to Fig. 9 and by way of comparison with Fig. 8, it is also seen that the number of retainers 1222, 1223 may be varied. The retainers may be substantially resilient or adapted for plastic deformation.

Still referring to Fig. 8, the clip 1110 is provided with other structure to facilitate stacking of the clips. The bridge 1116 of the clip 1110 includes upper and lower generally rectangular notches 1134, 1136, and the ends of the first and second arms each terminate in a narrowed rectangular projection 1162, 1164 sized to fit at the notches 1134, 1136 of a distally adjacent clip.

The clips according to the various embodiments of the invention are preferably made from a unitary piece of metal or metal alloy, such as titanium, titanium alloy, stainless steel, tantalum, platinum, other high Z (substantially radiopaque) materials, nickel-titanium alloy, martensitic alloy, or plastic, although other suitable biocompatible materials may be used.

The clips are particularly suitable for use in a flexible endoscopic clip applier, as described, though they may be also used in rigid instruments in both laparoscopic and open surgery. For endoscopic uses, the clips are preferably manufactured in the small sizes necessary for such minimally invasive procedures, e.g., a height of 0.04 - 0.12 inch (1 - 3 mm) between the outer surfaces of the first and second arms. For other uses, the clips may be appropriately sized. The clips, in all sizes, preferably, though not necessarily, have a length to height ratio of between approximately 2.5 and 5, and most preferably of approximately 3.4.

There have been described and illustrated herein several embodiments of a surgical clip and a method of using the same. While particular embodiments of the invention have been described, it is not intended that the invention be limited thereto, as it is intended that the invention be as broad in scope as the art will allow and that the specification be read likewise. Thus, while the clip is particularly adapted for manufacture in the small size necessary for flexible endoscopy, it will be appreciated that the clip may be made in other sizes as well. Furthermore, aspects of the various embodiments may be combined in yet other embodiments. That is, where structurally possible, the stacking structure of one embodiment may be combined with the retainer structure of another, and various retainer structure from one or more embodiments may be incorporated into a single clip. It will therefore be appreciated by those skilled in the art that yet other modifications could be made to the provided invention without deviating from its spirit and scope as claimed.

## Claims

1. A surgical clip, comprising:
a) a first arm having a first end and a bridge end;
b) a second arm having a retainer and a bridge end, said first and second arms defining a space therebetween, and said retainer extending from said second arm into said space; and
c) a bridge portion connecting said bridge ends of said first and second arms,
wherein said first and second arms and said bridge portion are in a generally U-shaped configuration.

2. A clip according to claim 1, wherein:
said retainer is substantially resilient.

3. A clip according to claim 1, wherein:
said retainer has a tissue piercing tip.

4. A clip according to claim 3, wherein:
said tissue piercing tip includes a barb.

5. A clip according to claim 1, wherein:
said retainer extends toward said first arm.

6. A clip according to claim 5, wherein:
said first arm defines a notch in communication with said space, and said retainer extends partially into said notch.

7. A clip according to claim 1, wherein:
said retainer is substantially straight.

8. A clip according to claim 1, wherein:
said retainer is curved.

9. A clip according to claim 8, wherein:
said clip defines an opening at an end opposite said bridge portion, and said retainer presents a concave surface toward said opening.

10. A clip according to claim 8, wherein:
said clip defines an opening at an end opposite said bridge portion, and said retainer presents a convex surface toward said opening.

11. A clip according to claim 1, wherein:
said first arm also includes a retainer extending into said space.

12. A clip according to claim 11, wherein:
at least one of said first and second arms includes a plurality of retainers.

13. A clip according to claim 12, wherein:
each of said first and second arms include a plurality of retainers, and said retainers on said first arm are interleaved with said retainers on said second arm.

14. A clip according to claim 1, wherein:
said second arm includes a second retainer.

15. A clip according to claim 14, wherein:
said second retainer is plastically deformable.

16. A clip according to claim 1, further comprising:
means for stacking said clip with a proximally adjacent clip and a distally adjacent clip.

17. A clip according to claim 16, wherein:
said means for stacking mechanically interlocks said clip with a proximally adjacent clip and a distally adjacent clip.

18. A clip according to claim 1, wherein:
said bridge portion includes at least one of a projection and a recess, and an interior portion of at least one of said first and second arms has a shape which corresponds to said at least one of said projection and said recess.

19. A clip according to claim 1, wherein:
said clip is made from titanium or a titanium alloy.

20. A clip according to claim 1, wherein:
said clip is made from one of stainless steel, tantalum, platinum, a radiopaque material, a nickel-titanium alloy, a martensitic alloy, and a plastic.

21. A clip according to claim 1, wherein:
said first and second arms and said bridge portion are substantially stiff.

22. A clip according to claim 1, wherein:
a dimension from an outer surface of said first arm to an outer surface of said second arm is 1 to 3 mm.

23. A clip according to claim 1, wherein:
a length to height ratio of said clip is between approximately 2.5 and 5.

24. A surgical clip, comprising:
a) a first arm having a first end and a bridge end;
b) a second arm having a first end and a bridge end, said first and second arms defining a space therebetween;
c) a bridge portion connecting said bridge ends of said first and second arms;
d) a retainer extending from one of said first and second arms into said space; and
e) means for stacking said clip with a proximally adjacent clip and a distally adjacent clip.

25. A clip according to claim 24, wherein:
said means for stacking mechanically interlocks said clip with a proximally adjacent clip and a distally adjacent clip.

26. A clip according to claim 24, wherein:
said means for stacking includes at least one of a projection and a recess on said bridge portion, and a shape which corresponding to said at least one of said projection and said recess on an interior portion of at least one of said first and second arms.

27. A clip according to claim 26, wherein:
said bridge portion includes an omega-shaped projection.

28. A clip according to claim 24, wherein:
said first and second arms are parallel and said retainer is resilient.

29. An arrangement of surgical clips, comprising:
a plurality of surgical clips mechanically interlocked in a linear arrangement.

30. An arrangement according to claim 29, wherein:
each said surgical clip includes a proximal projection and distal structure adapted to engage said proximal projection of a distally adjacent clip.

31. An arrangement according to claim 30, wherein:
one clip is detachable from said plurality of mechanically interlocked clips by application of a tensile force between said one clip and a remainder of said plurality of clips.

32. An arrangement according to claim 29, wherein:
each said clip includes,
i) a first arm having a first end and a bridge end,
ii) a second arm having a retainer and a bridge end, said first and second arms defining a space therebetween and said retainer extending from said second arm into said space, and
iii) a bridge portion connecting said bridge ends of said first and second arms.

33. An arrangement according to claim 32, wherein:
said retainer extends back toward said bridge portion and toward said first arm.

34. A method of applying a surgical clip to tissue, comprising:
a) providing a surgical clip having,
i) a first arm having a first end and a bridge end,
ii) a second arm having a retainer and a bridge end, said first and second arms defining a space therebetween and said retainer extending from said second arm into said space and at least partially blocking ingress into said space between said first and second arms, and
iii) a bridge portion connecting said bridge ends of said first and second arms; and
b) advancing said clip such that said first and second arms are pushed over tissue such that said retainer is bent by said tissue to permit movement of said clip over said tissue.

35. A method according to claim 34, further comprising:
c) prior to advancing said clip, compressing the tissue.

36. A method according to claim 35, further comprising:
d) providing a clip applier device,
wherein said compressing the tissue is performed by said clip applier device.
